# EUROPEAN PATENT SPECIFICATION

(11) **EP 2 774 913 B1**
(45) Date of publication and mention of the grant of the patent: **09.09.2015**
(21) Application number: 13158400.5
(22) Date of filing: 08.03.2013
(51) Int. Cl.: C07C 227/40, C07C 229/16, C11D 3/33

(54) **Process for working up an aqueous solution or slurry of a complexing agent**
Verfahren zur Aufbereitung von wässriger Lösung oder Schlamm eines Komplexbildners
Procédé de traitement d'une solution ou suspension aqueuse d'un agent complexant

(43) Date of publication of application: 10.09.2014
(73) Proprietor: BASF SE, 67056 Ludwigshafen (DE)
(72) Inventor: Hüffer, Stephan, 67063 Ludwigshafen (DE); Garcia Marcos, Alejandra, 67063 Ludwigshafen (DE); Hartmann, Markus, 67434 Neustadt (DE)

(56) References cited:
- US-A1- 2008 194 873

## Description

The present invention is directed towards a process for working up a reaction mixture selected from a aqueous solutions and aqueous slurries,, said reaction mixture containing at least one compound (C) selected from methylglycine diacetate, glutamic acid diacetate and from their respective salts, wherein the process according to the invention comprises the step of treating said reaction mixture with at least one inorganic compound (S) bearing at least one sulphur atom with an oxidation number in the range of from 2 to 4.

Organic complexing agents have numerous applications in which their capability for sequestering cations can be utilized. Organic complexing agents can be used for removing water hardness during, e.g., laundering or automatic dishwashing, and they can be used in formulations for hard surface cleaning. Particularly important examples of organic complexing agents are methyl glycine diacetate (MGDA) and glutamic acid diacetate (GLDA), in particular in form of their fully neutralized sodium salts. The synthesis of such compounds is still subject to current research and developmental work.

One problem emerging from many syntheses is that the products so obtained exhibit an unpleasant odour and a yellowing directly after said synthesis or after a certain time of storage. While not wishing to be bound by any theory, in many cases it appears that such unpleasant odour stems from ammonia. A very common synthesis for MGDA and GLDA applies a Strecker synthesis. An amino acid is being treated with acetaldehyde and ammonia. In particular, products from such Strecker syntheses exhibit a strong odour that is perceived as unpleasant. Products that stem from an alternative route based on alanine can develop a pungent odour as well.

It has been tried to remove the odour by, e. g., stripping with nitrogen or air/nitrogen combinations. Although a reduction of the odour can be observed the solution is not yet perfect. Other attempts have been to apply specific recrystallization technologies such as a vacuum cooling crystallization, see, e. g., US 2008/0194873. Although the latter technology is efficient to a certain extent it requires a lot of space.

It was therefore an objective of the present invention to provide a process for making a complexing agent that exhibits less strong odour or ideally no unpleasant odour, said process being feasible on industrial scale and not requiring a newly designed synthetic route to the respective compounds.

Accordingly, the process defined at the outset has been found, briefly also referred to as process according to the invention.

The process according to the invention starts with a reaction mixture, selected from aqueous solutions and aqueous slurries. Said reaction mixture contains at least one compound (C) selected from methylglycine diacetate, glutamic acid diacetate and from their respective salts, such as - but not limited to - their ammonium salts, their alkali metal salts, and mixed salts such as mixed alkali metal and alkaline earth metal salts, and mixed alkali metal ammonium salts. Salts of methylglycine diacetate and of glutamic acid diacetate can refer to partially neutralized and to fully neutralized respective compounds. Preferred are the potassium and the sodium salts of methylglycine diacetate and of glutamic acid diacetate, such as the monosodium salt of glutamic acid diacetate, the disodium salt of glutamic acid diacetate, the trisodium salt of glutamic acid diacetate, and the tetrasodium salt of glutamic acid diacetate. Even more preferred are the sodium salts of methylglycine diacetate such as the monosodium salt, the disodium salt and in particular the trisodium salt of MGDA, the latter also being abbreviated as MGDA-Na₃.

The respective aqueous solution or aqueous slurry may contain exactly one compound (C). In one embodiment of the present invention, the respective aqueous slurry or aqueous solution may contain two or even more compounds (C), preferably two compounds (C) either both derived from methylglycine diacetate or both derived from glutamic acid diacetate. For example, the respective aqueous solution may contain the disodium and the trisodium salt of methylglycine diacetate, or it may contain the trisodium and the tetrasodium salt of glutamic acid diacetate. In a preferred embodiment, the respective reaction mixture contains the tetrasodium salt of glutamic acid diacetate as the only compound (C), and in an even more preferred embodiment, respective reaction mixture contains the trisodium salt of MGDA as the only compound (C).

The respective reaction mixture may contain one or more organic products different from compound (C), especially one or more starting materials or intermediates or side product(s) stemming from the synthesis of methylglycine diacetate or of glutamic acid diacetate, for example partially hydrolyzed intermediates. Preferably, however, the share of starting materials and the like is very low, for example 1 % by weight, referring to the total solids content, or less, up to 100 ppm.

The respective reaction mixture may additionally contain ammonia, amines such as methyl amine, acetic acid or its respective salt, acetic aldehyde, alkali metal hydroxide, or organic solvents such as acetone or butanone.

Aqueous solutions containing at least one compound (C) are preferred. In a preferred embodiment, the process according to the invention is process for working up an aqueous solution containing at least one compound (C). In a preferred embodiment, working up (or work-up) will mean that the respective aqueous solution stems from the synthesis and already has undergone some work-up steps such as, but not limited to, stripping with nitrogen or nitrogen/air mixtures, or treatment with active charcoal, or UV treatment or crystallization.

The aqueous solution or slurry containing compound (C) may have a solids content in the range of from 5 to 92%, the solids content being determined by evaporation of the water and drying at 60°C until the weight remains constant. Therefore, ammonia cannot contribute to the solids content. For aqueous solutions, a solids content in the range of from 5 to 60% is preferred, and a solids content in the range of from 20 to 50% is even more preferred. For aqueous slurries, a solids content in the range of from 50 to 92% is preferred.

The process according to the invention is a process for working up a reaction mixture containing at least one compound (C), said reaction mixture being selected from aqueous solutions and aqueous slurries. The step defined in the outset and comprised in the process according to the invention is not necessarily the first or even the only work-up step. However, in the context of the present invention a reaction mixture has not yet undergone a formulation step such as crystallization or spray-drying.

Preferably, in the process according to the invention the step defined in the outset and comprised in the process according to the invention is being performed after having performed at least two of the following steps: stripping with nitrogen, filtering over charcoal and UV irradiation. Said reaction mixture selected from aqueous solutions and aqueous slurries may contain one or more organic solvents such as ethylene glycol or ethanol. Of all solvents in said aqueous solution or aqueous slurry, however, water constitutes at least 60 Vol.-% of the solvent phase. Preferable, said aqueous solution or aqueous slurry only contains traces, for example, 1 % by vol. or less of the solvent phase, is selected from one or more solvents.

The process according to the invention comprises the step of treating said aqueous slurry or preferably aqueous solution with at least one inorganic compound (S). Inorganic compound (S) is selected from compounds bearing at least one sulphur atom per molecule with an oxidation number in the range of from 2 to 4, preferably with an oxidation number of 4.

Examples of inorganic compounds (S) are sulfoxylates such as K₂SO₂ and Na₂SO₂. Examples of inorganic compounds (S) bearing at least one sulphur atom with an oxidation number of 4 are inorganic sulfites, disulfites, bisulfites, thiosulfates, and dithionites, and SO₂. Preferred are the salts of sodium, potassium, or ammonium salt of at least one anion selected from HSO₃⁻, SO₃²⁻, S₂O₃²-,S₂O₄²⁻, and of S₂O₅²⁻. Preferred are the sodium salts, NaHSO₃, Na₂SO₃, Na₂S₂O₃, Na₂S₂O₄, and Na₂S₂O₅. Particularly preferred are mixtures of sodium sulfite, Na₂SO₃, and sodium bisulfite, NaHSO₃. Such mixtures of Na₂SO₃ and NaHSO₃ may contain Na₂SO₃ and Na-HSO₃ in a molar ratio in the range of from 1 : 10 to 10 : 1, preferably in the range of from 1 : 2 to 2:1.

In a preferred embodiment of the present invention, the process according to the invention is being carried out in a way that said aqueous solution or aqueous slurry containing compound (C) is treated with gaseous SO₂, for example by sparging SO₂ through said aqueous solution or aqueous slurry.

In a more preferred embodiment of the present invention, the process according to the invention is being carried out in a way that said aqueous solution or aqueous slurry containing compound (C) is treated with a preferably aqueous solution of at least one sodium, potassium, or ammonium salt of at least one anion selected from HSO₃⁻, SOs²⁻, S₂O₃²⁻, S₂O₄²⁻, and of S₂O₅²⁻. In a preferred embodiment, said aqueous solution or aqueous slurry containing compound (C) is treated with a preferably aqueous solution of at least one sodium salt selected from NaHSO₃, Na₂SO₃, Na₂S₂O₃, Na₂S₂O₄, and Na₂S₂O₅. Particularly preferred is a treatment with an aqueous solution of a mixture of sodium sulfite, Na₂SO₃, and sodium bisulfite, NaHSO₃.

The concentration of said aqueous solution containing least one sodium, potassium, or ammonium salt of at least one anion selected from HSO₃⁻, SO₃²⁻, S₂O₃²⁻, S₂O₄²⁻, and of S₂O₅²⁻ can be chosen within a broad range, for example, 0.1 % by weight solutions up to saturated solutions can be applied.

Said treatment can be carried out by contacting a preferably aqueous solution containing at least one compound (S) with said aqueous slurry or to said aqueous solution containing at least one compound (C), for example by adding a preferably aqueous solution containing at least one compound (S) to said aqueous slurry or to said aqueous solution containing at least one compound (C).

In one embodiment, said treatment can be carried out in a stirred vessel or in a tubular reactor.

In one embodiment of the present invention, the process according to the invention is being carried out at a temperature in the range of from zero to 150°C, preferably in the range of from 20 to 100°C, even more preferably in the range of from 50 to 80°C. If it is desired to carry out the process according to the invention at a temperature above 100°C it is preferred to apply a pressure above normal pressure.

In one embodiment of the present invention, the process according to the invention is being carried out at a pressure in the range of from 500 mbar to 20 bar, normal pressure being preferred. In embodiments in which SO₂ is sparged through an aqueous solution or aqueous slurry of compound (C) it preferred to apply a pressure in the range of from 1.05 bar to 1.5 bar.

It is preferred that after having added compound (S), the resultant reaction mixture is allowed to react for 0.5 to 6 hours, preferred are 1 to 4 hours. In particular, it is preferred that after having added compound (S), the resultant reaction mixture is allowed to react for 0.5 to 6 hours at a temperature in the range of from 50 to 80°C, preferred are 1 to 4 hours at a temperature in the range of from 50 to 80°C.

In one embodiment of the present invention, it is preferred that 0.2 to 5.0 % by weight of compound (S) are added, referring to compound (C).

In one embodiment of the present invention said reaction mixture selected from aqueous solutions and aqueous slurries has a pH value in the range of from 5 to 14, preferably 7 to 13, determined at the beginning of the process according to the present invention. In embodiments that comprise the use of NaHSO₃ or SO₂, the pH value may drop about two units or less, for example 0.5 to 2 units.

In one embodiment of the present invention, the process according to the invention is being carried out in the absence of a catalyst.

In an alternative embodiment of the present invention, the process according to the invention in being carried out in the presence of a catalyst. Said catalyst can be heterogeneous or homogeneous, i. e. said catalyst can be selected from homogeneous catalysts such as dissolved catalytically active salts, and from heterogeneous catalysts. Preferably, said catalyst can be selected from metals and compounds containing at least one metal of the groups 8 to 10 of the periodic table of the elements.

A heterogeneous catalyst in the context with the present invention may be selected from:
(i) metal of the groups 8 to 10 of the periodic table of the elements containing catalysts supported on a solid support which is present in particulate form,
(ii) metal of the groups 8 to 10 of the periodic table of the elements containing catalysts supported on a solid support which is in non-particulate form, and
(iii) support-free catalytically active metals.

Within the context of the present invention, the term catalyst here comprises transition metal, which serves as catalytically active species ("main metal"), or optionally a precursor thereof, also optionally present support and optionally present doping.

"Solid support" or "support" is to be understood here as meaning those materials which are solid under the reaction conditions of the process according to the invention and which are appropriate for the shaping of the heterogeneous catalyst.

"Present in particulate form" is to be understood as meaning that the support in question in the form of particles, the average diameter of which is in the range from 0.1 µm to 2 mm, preferably 0.001 to 1 mm, preferably in the range from 0.005 to 0.5 mm, in particular 0.01 to 0.25 mm.

"Present in non-particulate form" is to be understood as meaning that the support has, in at least one dimension (width, height, depth), more than 2 mm, preferably at least 5 mm, where at least one further dimension, for example one or both further dimensions, can be less than 2 mm in size, for example in the range from 0.1 µm to 2 mm. In another variant, support present in non-particulate form has three dimensions which have a dimension of more than 2 mm, preferably at least 5 mm. A suitable upper limit is, for example, 10 m, preferably 10 cm.

Examples of supports which are present in non-particulate form are metal meshes, for example steel meshes or nickel meshes, also wires such as steel wires or nickel wires, also moldings, for example beads, Raschig rings, strands and tablets.

In one embodiment of the present invention, catalyst is used in the form of moldings, for example in the form of tablets or strands.

Examples of particularly suitable dimensions of moldings are tablets with dimensions 6.3 mm, 3.3 mm, 2.2 mm, and strands with a diameter in the range from 1.5 to 3 mm.

Examples of supports which are present in particulate form are powders, which may be free-flowing or suspended.

Examples of materials from which supports which are present in particulate form may be made are Al₂O₃, SiO₂, aluminosilicates, hydrotalcite, TiO₂, wood charcoal, in particular charcoal, Al₂O₃ or SiO₂.

Examples of support-free catalytically active metals (iii) are Raney metals, for example Raney-copper, Raney-nickel and Raney-cobalt. Support-free catalytically active metals can be present for example as sponge or skeletal catalysts.

Besides support and transition metal, the catalyst can comprise one or more molding agents, for example graphite or stearic acid.

Examples of particularly catalysts are transition metals of groups 9 or 10 in the oxidation state of zero, for example iridium, rhodium, platinum or especially palladium. Said catalysts are preferably supported on charcoal.

A very particularly preferred catalyst is supported palladium, with crystallites of Pd with an average diameter in the range of from 1 to 2.5 nm, and with a Pd content in the range of from 0.05 to 5 % by weight, referring to the catalyst as a whole, preferably in the range of from 0.2 to 3.5 % by weight. Examples of such catalysts are commercially available as Millipore® catalysts from Merck KGaA. Such catalysts can be activated by calcination in a stream of humid nitrogen, for examples at temperatures in the range of from 400 to 600°C. After activation, such Pd crystallites have an average diameter in the range of from 1 to 10 nm, preferably of 6 nm. The Pd crystallites may be finely dispersed on the inner and outer surface of the support material. Preferred support material is charcoal.

The process according to the invention can be combined with other measures to improve the quality of compound (C), such as, but not limited to recrystallizing, or treatment with active charcoal.

By performing the process according to the invention, aqueous solutions or slurries can be obtained that contain compound (C) and that have an advantageously reduced odour. This property will also be transferred to compound (C) when isolated, e. g., by spray-drying. Such advantageously reduced odour is not only detected by test personnel but also by gas chromatographic analysis of the head space of containers containing compound (C) for storage. In addition, by performing the process according to the invention a compound (C) can be obtained that exhibits a significantly improved storage behavior, also with respect to colour stability, with and without other ingredients useful for hard surface cleaning and in particular with beaching agents such as sodium percarbonate.

Although the process according to the invention can advantageously be applied to aqueous solutions containing at least one compound (C) stemming from a Strecker synthesis it is also useful for aqueous solutions or aqueous slurries stemming from synthetic routes different from a Strecker synthesis such as, but not limited to, an oxidation of bisethoxylated alanine.

The present invention is further illustrated by the following non-limiting examples. General remarks: colour numbers refer to Hazen colour numbers unless expressly indicated otherwise.

### I. Manufacture of a reaction mixture containing the trisodium salt of MGDA

An amount of 148 g (1.0 mol) of pure methyl glycine diacetonitrile was introduced at 25°C with vigorous stirring into a flask containing 608 of 20% by weight sodium hydroxide g (3.04 mol) solution within two hours. Subsequently, the mixture was stirred further under nitrogen first at 30°C for 3 h and then at 40°C for 2 h. Thereafter, the mixture was stripped with nitrogen at 95°C for 2 hours. During stripping, the solids concentration was kept below 45% by weight by adding water. The stripping resulted in a yellow-orange solution (Hazen color number: 140) with the following composition: MGDA-Na₃: 260 g (0.96 mol, yield = 96%), corresponding to 650 g of a 40% by weight MGDA-Na₃ solution; NTA-Na₃: < 0.1% by weight; iminodiacetate-Na₂: 0.8% by weight; Na₂CO₃: 0.1% by weight; NaOH: 0.2% by weight; Na acetate: 0.6% by weight; Na formate: 0.07% by weight; Na glycolate: 0.07% by weight; Na lactate: 0.0% by weight; Na glycinate: 0.06% by weight; Na alaninate: 0.1 % by weight; acetaldehyde: 230 ppm; water: 55% by weight. The aqueous solution so obtained had a pungent odour from acetaldehyde and ammonia.

### II. Working up of a solution containing MGDA-Na₃

### II.1 General operation procedure for examples according to the invention

The experiment from I. was repeated in order to provide more aqueous solution containing MGDA-Na3. A 2-I-flask with stirrer and thermometer was charged with 1 kg aqueous solution of step I. The aqueous solution was heated to 60°C under stirring. At 60°C, an amount of inorganic compound (S) was added according to table 2. In cases where inorganic compound (S) was SO₂, gaseous SO₂ was introduced through a tube at a speed of 3 NI/h (NI: liter under normal pressure and 20°C).

As soon as the amount of compound (S) as specified in table 2 had been added, the resultant mixture was heated to 75°C under stirring. The resultant mixture was stirred und kept at a temperature in the range of from 75 to 80°C for 4 hours. Then, the resultant solution was allowed to cool down to 25°C.

### II.2 General operation procedure for comparative examples

The experiment from I. was repeated in order to provide more aqueous solution containing MGDA-Na3. A 2-I-flask with stirrer and thermometer was charged with 1 kg aqueous solution of step I. The aqueous solution was first heated to 60°C and then to 75°C under stirring but no compound (S) was added. Instead, nitrogen 3 NI/h was bubbled through the solution. The resultant mixture was stirred und kept at a temperature in the range of from 75 to 80°C for 4 hours. Then, the resultant solution was allowed to cool down to 25°C.

### II.3 Working up in the presence of a catalyst

A catalyst (Pd on charcoal) commercially available as Millipore® catalysts from Merck KGaA was activated as follows. The commercially available catalyst was calcined in a stream of humid nitrogen (bubbling through water of 25°C), calcination temperature of 500, for 10 hours. After activation, such Pd crystallites had an average diameter of 6 nm. The Pd crystallites were finely dispersed on the inner and outer surface of the charcoal.

The activated catalyst was added to the solution according to example II.1 together with the addition of compound (S).

Alternatively, in a so-called after treatment, a column (inner diameter: 8.9 cm, height: 45 cm) was charged with activated catalyst, height of catalyst area: 20 cm. Solution according to general procedure II.1 was stirred for only 20 min after addition of compound (S) and then allowed to pass the catalyst (from top to bottom), residence time in the catalyst area: 5 minutes.

### III. Test results

### III.1 Head space

A 500 ml container was charged with an amount of 100 ml of solution according to the invention (or of comparative solution). After 1 hour, the odour was checked by a test team, and grades from 1 (very good) to 5 (inacceptable) were awarded. In addition, the head space of said container was analyzed by gas chromatography for ammonia and acetaldehyde.

### III.2 Storage tests

A 500 ml container was charged with an amount of 100 ml of solution according to the invention (or of comparative solution) and shaken in a sample shaker for 2 weeks at 60°C. Then, resultant solution was allowed to cool down to 25°C, and its colour number was determined.

### III.3 Spray-Drying of MGDA solutions

The procedure below was carried out in a fluid-bed spray granulator that was equipped with a cyclone, a filter, and a gas scrubber.

The respective solution worked up according to the invention, or a comparative solution was heated to 90°C with continuous and intense stirring and was used at this temperature for the granulation. Under the following conditions, a steady state granulating operation was achieved:
Feed air temperature: 125°C
Departing air temperature: 65°C
Granule temperature: 65 to 70°C
Intake air volume: 1300 m³/h
Spraying air temperature: 90°C, spraying air volume: 3 bar

In order to raise the crystallinity, the product prepared was after-treated with a temperature profile commencing with a bed temperature of 70°C, then raising this temperature to around 110 - 120°C over the course of around an hour, and subsequently holding at this temperature for around 60 minutes.

Screening at 1000 microns and re-use of ground coarse material as crystallization seeds for the granulation process led to a stable operation with a yield of about 20 kg of granules per hour in the desired quality.

The granules had diameters in the range of from 0.5 to 1 mm (share: 70% by weigh or more).

### III.4 Manufacture of a gel for automatic dishwashing and storage test

The following ingredients were mixed according the order in table 1. A 1-I-beaker was charged with test solution according to example II. Then, the other ingredients according to table 1 were added under magnetic stirring at room temperature. Then, balance to 100 ml was added. Test gels were obtained.

**Table 1: composition of test gels for automatic dishwash**

| Ingredient | g tq./ 100 g | active [g/100 g] |
|---|---|---|
| Aqueous solution according to the invention or comparative solution (40% solids) | 37.50 | 15.00 |
| Na-citrate, dihydrate | 5.00 | 5.00 |
| Polymer A solution | 10.20 | 5.00 |
| D-Sorbitol | 5.00 | 5.00 |
| Non-ionic surfactant | 2.00 | 2.00 |
| Citric acid solution (50% am) | 2.22 | 1.11 |
| Xanthan Gum | 0.40 | 0.40 |
| Savinase Ultra 16 XL | 4.00 | 0.40 |
| Stainzyme® 12 L | 2.00 | 0.20 |
| De-ionized water | 31.68 | balance to 100 |
| Total | 100.00 | 100.00 |

Savinase ultra 16 XL is a serine-type protease from bacillus sp., commercially available from Novozymes. Stainzyme® 12 L is a liquid amylase.
Polymer A solution: 49 % by weight aqueous solution of polyacrylic acid, partially neutralized with NaOH, pH value of the solution: 3.7 (upon 1:10 dilution with water), average molecular weight M_{w}: 4,000 g/mol
Non-ionic surfactant:
tq.: tel quel 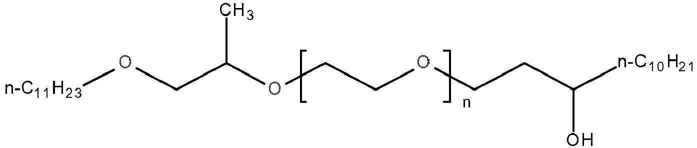 with the integer n being 22.

The test gels were stored over a period of 3 weeks at 37°C. After such storage, the colour numbers of the test gels were determined.

### III.5 Manufacture of a powder for automatic dishwashing and storage test

30 g of granules according to III.3 were mixed with 15 g sodium percarbonate (2 Na₂CO₃·3 H₂O₂) and stored at 37°C at a humidity of 70% for 2 weeks. After those two weeks, the resultant ADW powder was dissolved in 60 ml of water, and the colour number was determined.

The results are summarized in table 2. All colour numbers are according to Hazen. The Hazen colour number can be determined according to DIN EN ISO 6271-1 or 6271-2.

**Table 2: Experimental parameters and test results**

| (S) | (S) [g] | Acetaldehyde [ppm] | NH₃ [ppm] | Odour [grade] | Hazen Colour No. III.2 | Hazen Colour No. III.4 | Hazen Colour No. III.5 |
|---|---|---|---|---|---|---|---|
| NaHSO₃ | 5 | 18 | 3 | 2 | 120 | 210 | 220 |
| NaHSO₃ | 20 | 4 | < 1 | 2 | 78 | 130 | 180 |
| NaHSO₃(*) | 10 | 3 | < 1 | 2 | 60 | 122 | 145 |
| Na₂S₂O₅ | 7 | 8 | 5 | 2 | 86 | 200 | 260 |
| Na₂S₂O₅ | 30 | 3 | < 1 | 2 | 59 | 110 | 168 |
| SO₂ | 2 | 13 | < 1 | 2 | 86 | 190 | 245 |
| SO₂ | 5 | 4 | < 1 | 1 | 56 | 155 | 196 |
| SO₂ | 12 | 2 | < 1 | 2 | 40 | 123 | 161 |
| SO₂(**) | 5 | 2 | < 1 | 1 | 35 | 104 | 150 |
| Na₂SO₃ | 5 | 22 | 4 | 3 | 70 | 165 | 193 |
| Na₂SO₃ | 12 | 16 | 6 | 2 | 52 | 108 | 175 |
| Na₂S₂O₄ | 5 | 120 | 11 | 4 | 93 | 203 | 205 |
| Na₂S₂O₃ | 5 | 90 | 9 | 3 | 76 | 194 | 264 |
| none | - | 230 | 12 | 5 | 180 | 290 | 345 |

| | | | | | | | |
|---|---|---|---|---|---|---|---|
| (*): after-treatment with catalyst (**) in presence of catalyst <1: not detectable within experimental error | | | | | | | |

## Claims

1. Process for working up a reaction mixture selected from a aqueous solutions and aqueous slurries, said reaction mixture containing at least one compound (C) selected from methylglycine diacetate, glutamic acid diacetate and from their respective salts, wherein the process comprises the step of treating said reaction mixture with at least one inorganic compound (S) bearing at least one sulphur atom with an oxidation number in the range of from 2 to 4.

2. Process according to claim 1, **characterized in that** inorganic compound (S) is selected from inorganic sulfoxylates, SO₂, sulfites, disulfites, bisulfites, thiosulfates, and dithionites.

3. Process according to claim 1 or 2, **characterized in that** compound (C) is selected from sodium salts of methylglycine diacetate.

4. Process according to any of the preceding claims wherein said aqueous solution or aqueous slurry containing compound (C) is treated with gaseous SO₂.

5. Process according to any of the preceding claims, **characterized in that** said reaction mixture is treated with a solution of the sodium, potassium, or ammonium salt of at least anion selected from HSO₃⁻, SO₃²⁻, S₂O₄²⁻, or S₂O₅²⁻.

6. Process according to any of the preceding claims, **characterized in that** said treatment is being carried out in the presence of a catalyst.

7. Process according to claim 6, **characterized in that** said catalyst may be heterogeneous or homogeneous, and that said catalyst is selected from metals and compounds comprising at least one metal of the groups 8 to 10 of the periodic table of the elements.

8. Process according to any of the proceeding claims, **characterized in that** it is a process for working up an aqueous solution containing at least one compound (C).

9. Process according to any of the preceding claims, **characterized in that** it is carried out at a temperature in the range of from zero to 150°C.

10. Process according to any of the preceding claims, **characterized in that** the solids content of said reaction mixture is in the range of from 5 to 92%.

11. Process according to any of the preceding claims, **characterized in** said reaction mixture is treated with a solution containing NaHSO₃ and Na₂SO₃.

12. Process according to any of the preceding claims, **characterized in that** said reaction mixture has a pH value in the range of from 5 to 14.

## Patentansprüche

1. Verfahren zur Aufarbeitung einer aus wässrigen Lösungen und wässrigen Aufschlämmungen ausgewählten Reaktionsmischung, die mindestens eine Verbindung (C), die aus Methylglycindiacetat, Glutaminsäurediacetat und deren jeweiligen Salzen ausgewählt ist, enthält, bei dem man die Reaktionsmischung mit mindestens einer anorganischen Verbindung (S) mit mindestens einem Schwefelatom mit einer Oxidationszahl im Bereich von 2 bis 4 behandelt.

2. Verfahren nach Anspruch 1, **dadurch gekennzeichnet, dass** die anorganische Verbindung (S) aus anorganischen Sulfoxylaten, SO₂, Sulfiten, Disulfiten, Bisulfiten, Thiosulfaten und Dithioniten ausgewählt wird.

3. Verfahren nach Anspruch 1 oder 2, **dadurch gekennzeichnet, dass** Verbindung (C) aus Natriumsalzen von Methylglycindiacetat ausgewählt wird.

4. Verfahren nach einem der vorhergehenden Ansprüche, bei dem die wässrige Lösung bzw. wässrige Aufschlämmung, die Verbindung (C) enthält, mit gasförmigem SO₂ behandelt wird.

5. Verfahren nach einem der vorhergehenden Ansprüche, **dadurch gekennzeichnet, dass** die Reaktionsmischung mit einer Lösung des Natrium-, Kalium- oder Ammoniumsalzes von mindestens einem aus HSO₃⁻, SO₃²⁻, S₂O₄²⁻ oder S₂O₅²⁻ ausgewählten Anion behandelt wird.

6. Verfahren nach einem der vorhergehenden Ansprüche, **dadurch gekennzeichnet, dass** die Behandlung in Gegenwart eines Katalysators durchgeführt wird.

7. Verfahren nach Anspruch 6, **dadurch gekennzeichnet, dass** der Katalysator heterogen oder homogen sein kann und dass der Katalysator aus Metallen und Verbindungen mit mindestens einem Metall der Gruppen 8 bis 10 des Periodensystems der Elemente ausgewählt wird.

8. Verfahren nach einem der vorhergehenden Ansprüche, **dadurch gekennzeichnet, dass** es sich um ein Verfahren zum Aufarbeiten einer wässrigen Lösung, die mindestens eine Verbindung (C) enthält, handelt.

9. Verfahren nach einem der vorhergehenden Ansprüche, **dadurch gekennzeichnet, dass** es bei einer Temperatur im Bereich von null bis 150°C durchgeführt wird.

10. Verfahren nach einem der vorhergehenden Ansprüche, **dadurch gekennzeichnet, dass** der Feststoffgehalt der Reaktionsmischung im Bereich von 5 bis 92% liegt.

11. Verfahren nach einem der vorhergehenden Ansprüche, **dadurch gekennzeichnet, dass** die Reaktionsmischung mit einer NaHSO₃ und Na₂SO₃ enthaltenden Lösung behandelt wird.

12. Verfahren nach einem der vorhergehenden Ansprüche, **dadurch gekennzeichnet, dass** die Reaktionsmischung einen pH-Wert im Bereich von 5 bis 14 aufweist.

## Revendications

1. Procédé de traitement final d'un mélange réactionnel choisi parmi les solutions aqueuses et les suspensions aqueuses, ledit mélange réactionnel contenant au moins un composé (C) choisi parmi le diacétate de méthylglycine, le diacétate d'acide glutamique et leurs sels respectifs, le procédé comprenant l'étape de traitement dudit mélange réactionnel avec au moins un composé inorganique (S) portant au moins un atome de soufre avec un nombre d'oxydation dans la gamme de 2 à 4.

2. Procédé selon la revendication 1, **caractérisé en ce que** le composé inorganique (S) est choisi parmi les sulfoxylates inorganiques, SO₂, les sulfites, les disulfites, les bisulfites, les thiosulfates, et les dithionites.

3. Procédé selon la revendication 1 ou 2, **caractérisé en ce que** le composé (C) est choisi parmi les sels de sodium de diacétate de méthylglycine.

4. Procédé selon l'une quelconque des revendications précédentes dans lequel ladite solution aqueuse ou suspension aqueuse contenant le composé (C) est traitée avec du SO₂ gazeux.

5. Procédé selon l'une quelconque des revendications précédentes, **caractérisé en ce que** ledit mélange réactionnel est traité avec une solution du sel de sodium, potassium ou ammonium d'au moins un anion choisi parmi HSO₃⁻, SO₃²⁻ S₂O₄²⁻ et S₂O₅²⁻.

6. Procédé selon l'une quelconque des revendications précédentes, **caractérisé en ce que** ledit traitement est réalisé en présence d'un catalyseur.

7. Procédé selon la revendication 6, **caractérisé en ce que** ledit catalyseur peut être hétérogène ou homogène, et **en ce que** ledit catalyseur est choisi parmi les métaux et les composés comprenant au moins un métal des groupes 8 à 10 du tableau périodique des éléments.

8. Procédé selon l'une quelconque des revendications précédentes, **caractérisé en ce qu'**il s'agit d'un procédé de traitement final d'une solution aqueuse contenant au moins un composé (C).

9. Procédé selon l'une quelconque des revendications précédentes, **caractérisé en ce qu'**il est réalisé à une température dans la gamme de zéro à 150 °C.

10. Procédé selon l'une quelconque des revendications précédentes, **caractérisé en ce que** la teneur en matières solides dudit mélange réactionnel se situe dans la gamme de 5 à 92 %.

11. Procédé selon l'une quelconque des revendications précédentes, **caractérisé en ce que** ledit mélange réactionnel est traité avec une solution contenant du NaHSO₃ et du Na₂SO₃.

12. Procédé selon l'une quelconque des revendications précédentes, **caractérisé en ce que** ledit mélange réactionnel a une valeur de pH dans la gamme de 5 à 14.
